# FASCICULE DE BREVET EUROPEEN

(11) **EP 0 839 206 B1**
(45) Date de publication et mention de la délivrance du brevet: **12.09.2001**
(21) Numéro de dépôt: 96925809.4
(22) Date de dépôt: 17.07.1996
(51) Int. Cl.: C12N 15/86, C12N 5/10, A61K 48/00

(54) **SEQUENCES REGULATRICES ISSUES DU GENE CD4 ET A EXPRESSION SPECIFIQUE DANS LES LYMPHOCYTES T MATURES**
GENREGULATORISCHE SEQUENZEN VON CD4, WELCHE SPEZIFISCH IN REIFEN T-ZELLEN EXPRIMIERT WERDEN
CD4 GENE REGULATORY SEQUENCES SPECIFICALLY EXPRESSED IN MATURE T CELLS

(30) Priorité: 17.07.1995 FR 9508616
(43) Date de publication de la demande: 06.05.1998
(73) Titulaire: UNIVERSITE PIERRE ET MARIE CURIE PARIS VI, 75252 Paris Cédex 05 (FR)
(72) Inventeur: KLATZMANN, David, F-75013 Paris (FR); SALMON, Patrick, CA 94530 (US); BOYER, Olivier, F-75018 Paris (FR)
(74) Mandataire: Becker, Philippe
(86) Numéro de dépôt international: FR9601122
(87) Numéro de publication internationale: WO9704118

(56) Documents cités:
- MOLECULAR AND CELLULAR BIOLOGY, vol. 14, no. 2, Février 1994, pages 1084-1094, XP002001074 ZAHER HANNA ET AL.: "Specific expression of the human CD4 gene in mature CD4+CD8- and immature CD4+CD8- T cells and in macrophages of transgenic mice"
- EMBO JOURNAL, vol. 12, no. 4, 1993, EYNSHAM, OXFORD GB, pages 1547-1553, XP002001075 NIGEL KILLEEN ET AL.: "Regulated expression of human CD4 rescues helper T cell development in mice lacking expression of endogenous CD4" cité dans la demande
- PROCEEDINGS OF THE NATIONAL ACADEMY OF SCIENCES OF USA, vol. 90, no. 16, 15 Août 1993, WASHINGTON US, pages 7739-7743, XP002001076 PATRICK SALMON ET AL.: "Characterization of the human CD4 gene core promoter is tissue-specific and is activated by Ets proteins " cité dans la demande
- MOLECULAR AND CELLULAR BIOLOGY, vol. 11, no. 11, 1 Novembre 1991, pages 5506-5515, XP000567480 SHINICHIRO SAWADA ET AL.: "Identification and characterization of a T-cell-specific enhancer adjacent to the murine CD4 gene" cité dans la demande
- FASEB JOURNAL FOR EXPERIMENTAL BIOLOGY, vol. 8, no. 5, 18 Mars 1994, BETHESDA, MD US, page A785 XP002001077 MCCREADY P.M. ET AL.: "Transient transfection of T cell lines with the murine CD4 promoter and the T cell-specific enhancer"
- THE JOURNAL OF IMMUNOLOGY, vol. 156, no. 5, 1 Mars 1996, pages 1873-1879, XP002016452 PATRICK SALMON ET AL.: "characterization of a intronless CD4 minigene expressed in mature CD4 and CD8 T cells, but not expressed in immature thymocytes"

## Description

La présente invention est relative à l'utilisation de séquences régulatrices issues du gène CD4 dans des vecteurs d'expression d'un gène hétérologue ou d'un transgène, leur conférant, quand ils sont intégrés dans des cellules du système hématopoïétique et notamment les cellules souches, ou plus généralement les cellules de la moelle osseuse, une expression spécifique au niveau des lymphocytes T matures à l'exclusion des lymphocytes T immatures, et notamment après la sélection du répertoire.

Au cours de la maturation des cellules T, l'expression des récepteurs CD4 et CD8 répond à un mécanisme complexe de régulation ; l'expression différentielle des glycoprotéines CD4 et CD8 est couplée au choix de l'une des voies de la différentiation soit en lymphocytes T auxiliaires soit en lymphocytes T cytotoxiques ; ainsi les thymocytes, c'est-à-dire les cellules hématopoïétiques engagées dans une voie de différentiation T, ont tout d'abord un phénotype CD4- CD8-(doubles négatifs ou DN), puis ils acquièrent l'expression conjointe de la molécule CD4 et de la molécule CD8 pour former les thymocytes doubles positifs (DP) CD4+ CD8+ et enfin, cette population se différencie en lymphocytes simples positifs (SP) qui expriment soit CD4 pour les lymphocytes T auxiliaires soit CD8 pour les lymphocytes T cytotoxiques. Les thymocytes qui se lient aux molécules d'histocompatibilité de classe I deviendront des lymphocytes T cytotoxiques CD4- CD8+, alors que ceux qui se lient aux molécules de classe Il deviendront des lymphocytes T auxiliaires CD4+ CD8-; après ce processus intra-thymique de sélection du répertoire, les thymocytes quittent le thymus et atteignent le système périphérique : sang et organes lymphoïdes.

Une synthèse de ce processus de différenciation est décrite dans Nicolic-Zugic, J, (1991) Immunol. Today 12 : 65-70.

La régulation de l'expression du gène CD4 est actuellement étudiée par de nombreuses équipes, l'élucidation d'un tel mécanisme pouvant aider à la compréhension du contrôle des développements des cellules T.

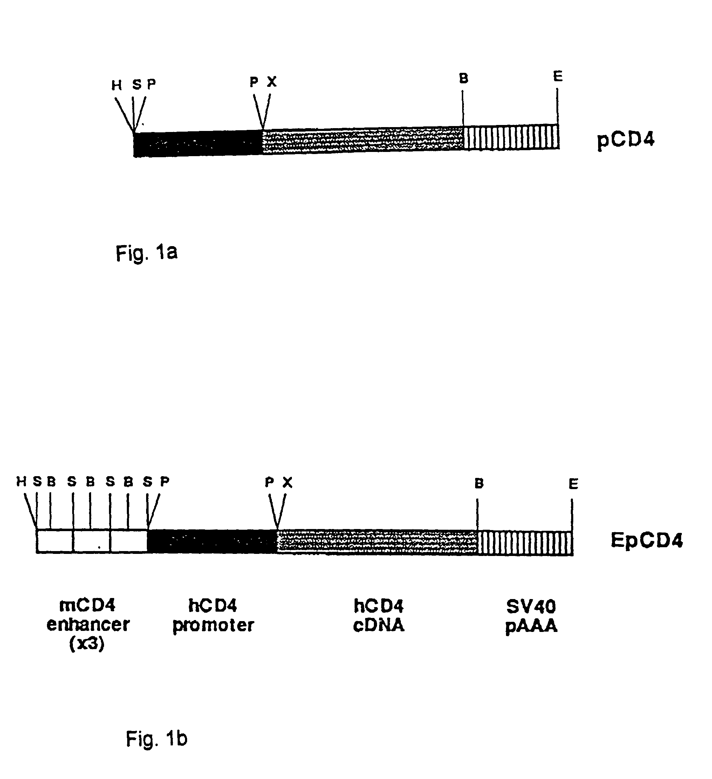

Plusieurs équipes travaillent sur les séquences régulatrices du gène CD4 humain ou murin. Parmi les plus récents, il convient de citer les travaux suivants : Killeen et al (EMBO Journal vol 12 n°4 p1547, 1993) ont montré qu'un transgène portant le CD4 humain d'une taille d'environ 35 kb possédait toutes les séquences génomiques nécessaires au contrôle de l'expression au cours du développement dans des souris transgéniques. Dans ce fragment, deux éléments de régulation distincts ont été identifiés comme critiques pour l'expression du transgène : le premier est une séquence amplificatrice, ou amplificateur (pour enhancer), située soit 13 kb en amont du site cap du CD4 murin, soit 6 kb en amont du CD4 humain. Blum M.D. et al (J. Exp. Med. (1993) 177 n° 5 : 1343-1358) ont identifié et séquencé l'amplificateur du CD4 humain, et Sawada et al. (Mol. Cell. Biol; 11 55: 5506-5515, 1991) ont identifié et séquencé l'amplificateur du CD4 murin. Ces deux équipes ont montré que l'expression de cette amplificateur est spécifique du gène CD4 dans les lymphocytes T, qu'ils soient matures ou immatures ; P. Salmon et al, (Proc. Natl. Acad. Sci., USA 90 : 7739-7743 (1993)) ont analysé la structure et la séquence du promoteur du CD4 humain et l'ont comparé avec celui du CD4 murin. Ils ont identifié un fragment d'environ 1100 paires de bases présentant la fonction de promoteur spécifique du CD4. Un alignement de cette séquence avec celle du promoteur CD4 murin indique une structure très similaire comme le montre la figure 1 de ce même article.

La différenciation des lymphocytes T met en oeuvre d'autres éléments de régulation de type silenceur (silencer) dont la présence conduit à la diminution ou à l'arrêt de la transcription des gènes quand ils sont dans leur voisinage. Sawada et al, dans Cell 77 : 911-929, (1994) ont mis en évidence l'existence d'un silenceur dans un premier intron du gène CD4, et dont une des fonctions est l'extinction de la transcription du gène CD4 dans les lymphocytes T matures CD8+.

Enfin le gène du CD4 humain a été analysé par Z. Hanna et al. (Mol. and Cell. Biology (1994) p. 1084-1094) dans une construction qui comprend au moins 3 introns d'une longueur totale de 12Kb.

Toutes les études récentes, citées plus haut, indiquent que l'expression du gène CD4 est contrôlée de manière similaire dans les cellules humaines et murines ; néanmoins les relations entre les différents éléments de régulation et leurs fonctions n'est pas élucidées.

La présente invention résulte de la découverte faite par les inventeurs d'une combinaison de séquences régulatrices qui présentent des propriétés inattendues et totalement imprévisibles : cette combinaison composée d'éléments génétiques issus des séquences en 5' du gène CD4, le cas échéant combinés avec le cDNA du gène CD4, a son expression restreinte aux cellules T matures et aux cellules NK dans un modèle de souris transgénique ; aucune expression n'a pu être détectée dans les cellules T immatures incluant les thymocytes doubles positifs ou doubles négatifs. Cet ensemble de séquences régulatrices est le premier à avoir cette spécificité d'expression ce qui est particulièrement important notamment pour une utilisation dans le cadre de la thérapie génique, pour des programmes qui nécessiteraient l'expression spécifique d'un gène dans les lymphocytes T matures.

Rien, dans l'état de la technique, ne laissait supposer qu'une telle combinaison pouvait conduire à ce résultat.

La présente invention résulte de la découverte selon laquelle la combinaison de l'amplificateur et du promoteur de CD4 promeut l'expression d'un gène « reporter» , en l'occurrence le cDNA du gène CD4 humain, dans des thymocytes matures de souris alors qu'il n'est pas exprimé dans des doubles positifs CD4+ CD8+ immatures ; ce résultat est surprenant en ce sens qu'il implique l'existence d'un élément de régulation supplémentaire non encore connu et qui permettrait l'expression du gène CD4 au stade immature ; cet élément qui agirait en « cis » serait absent de la construction puisqu'aucun élément de régulation de la souris qui pourrait agir en trans n'est efficace pour une telle expression dans les cellules immatures.

Un résultat prévisible est que l'association à cette combinaison du silenceur décrit par Sawada et al, 1994 (voir supra) constitue une cassette d'expression d'un gène hétérologue exclusivement dans les cellules T auxiliaires SP CD4+ CD8-.

La présente invention porte sur un système d'expression d'une protéine ou d'un gène hétérologue comprenant un vecteur recombinant, utilisable pour la transfection des cellules de la lignée hématopoïétique, notamment les cellules souches du sang ou de la moelle, de telle façon que la cellule ainsi tranduite n'exprimera la protéine ou le gène hétérologue portée par le vecteur que dans les cellules T matures après la sélection du répertoire, et ce à l'exclusion d'une expression dans des cellules T immatures notamment les cellules CD4- CD8- et CD4+ CD8+ ; ledit vecteur du système d'expression est pourvu de toutes les séquences nécessaires à son expression et il est caractérisé en ce qu'il contient au moins un amplificateur d'un gène CD4 issu de la même espèce ou d'une espèce différente.

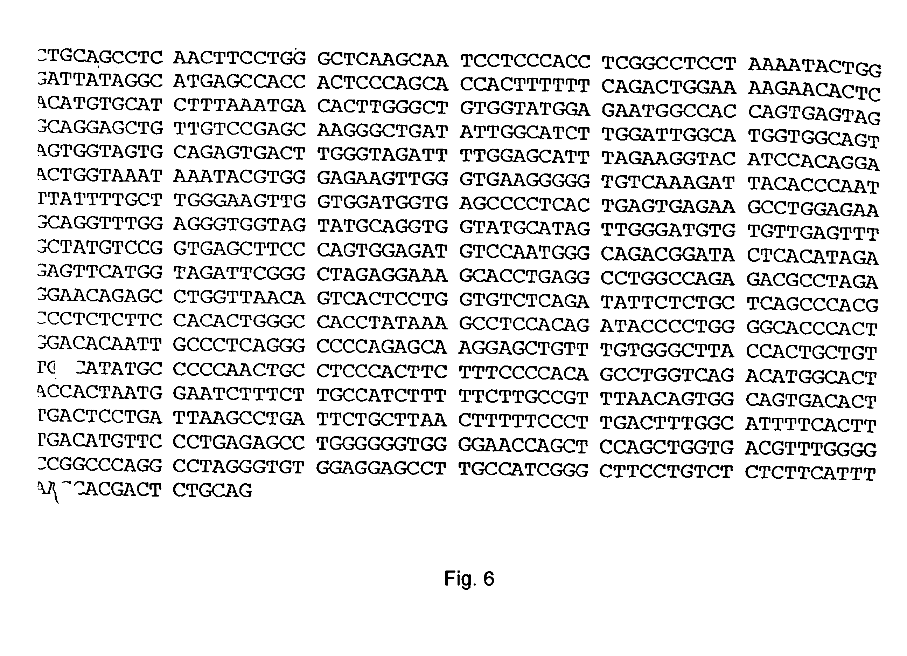

Le vecteur du système d'expression peut contenir également, combiné à l'amplificateur, un promoteur constitué de l'une des séquences suivantes :
- le promoteur d'un gène CD4, notamment le promoteur du gène CD4 humain tel que représenté dans la figure 6, ou
- la séquence comprise entre les nucléotides -498 et +16 dans la numératation de P. SALMON (1993 supra) (correspondant aux nucléotides 579 à 1092 de la figure 6), ou,
- la séquence comprise entre les nucléotides -165 et +16 (P. SALMON, 1993 supra) (correspondant aux nucléotides 912 à 1092 de la figure 6) ou,
- toute séquence dérivée de l'une des séquences précédentes par addition, délétion ou substitution de nucléotides sans modification substantielle de l'expression de la protéine hétérologue sous le contrôle de ce promoteur.

Un exemple particulier d'amplificateur performant dans le cadre de cette invention est celui du CD4 murin et constitué de 339 ou tout ou partie des 339 paires de bases et décrit dans Sawada et al (1991) cité plus haut, cet amplificateur pouvant être présent soit sous forme d'une séquence simple soit sous forme d'un séquence répétée de deux à cinq fois et dont la séquence est la suivante :

TGTTGGGGTT CAAATTTGAG CCCCAGCTGT TAGCCCTCTG
CAAAGAAAAA AAAAAAAAAA AAAGAACAAA GGGCCTAGAT
TTCCCTTCTG AGCCCCACCC TAAGATGAAG CCTCTTCTTT
CAAGGGAGTG GGGTTGGGGT GGAGGCGGAT CCTGTCAGCT
TTGCTCTCTC TGTGGCTGGC AGTTTCTCCA AAGGGTAACA
GGTGTCAGCT GGCTGAGCCT AGGCTGAACC CTGAGACATG
CTACCTCTGT CTTCTCATGG CTGGAGGCAG CCTTTGTAAG
TCACAGAAAG TAGCTGAGGG GCTCTGGAAA AAAGACAGCC
AGGGTGGAGG TAGATTGGT

L'amplificateur du CD4 humain, décrit par M.D. Blum et al (cité plus haut) entre dans le champs de l'invention, qu'il soit utilisé seul ou en combinaison avec le promoteur du CD4.

Par le terme amplificateur d'un gène CD4, on entend non seulement les séquences décrites par Sawada et al ou par Blum et al, mais également d'autres séquences dérivées d'autres espèces de mammifères et présentant un degré d'homologie suffisant avec les séquences ci-dessus comprenant notamment, par exemple, les séquences spécifiques de protéines nucléaires CD4-1, CD4-2 et CD4-3 décrits dans Sawada et al, (1991).

L'implication de cette propriété particulière - non expression dans les cellules T immatures, expression dans les cellules T matures-fait apparaître les avantages de la construction de l'invention pour son incorporation dans des médicaments utilisables en thérapie génique dans toutes les indications où l'expression de transgène dans les cellules T pourrait être un moyen pour traiter ou prévenir certaines pathologies ou dysfonctionnements notamment les infections virales, notamment par le VIH, des pathologies de l'expression de certains gènes dans des lymphocytes T par exemple le déficit en adénosine désaminase ou plus généralement les déficits immunitaires, primitifs ou secondaires, les pathologies auto-immunes ou les greffes. Cependant le thérapeute se heurte à une difficulté qui est la suivante : si les lymphocytes T matures sont pris comme cibles en thérapie génique, il est obligé de réaliser un transfert de gènes dans ces lymphocytes T matures ex vivo et ces derniers doivent être au préalable mis en culture pour effectuer ce transfert par les vecteurs appropriés ; après réinjection leur demi-vie est relativement courte ; au contraire si on choisit comme stratégie de cibler les cellules souches hématopoïétiques qui donnent naissance après différentiation aux lymphocytes T on se heurte alors à deux problèmes différents : le premier est celui de l'expression du gène d'intérêt dans les cellules souches qui n'est pas toujours souhaitable, et le deuxième est celui de la perturbation possible induite par l'expression du gène hétérologue dans les cellules immatures sur la sélection du répertoire dans le thymus.

La construction de l'invention permet de surmonter ce double inconvénient par un transfert aux cellules souches hématopoïétiques d'un gène hétérologue qui ne sera exprimé que postérieurement à la sélection du répertoire.

Le transfert de gènes dans les cellules hématopoïétiques a comme il a été dit plus haut été envisagé pour l'expression d'une protéine d'intérêt de type adénosine désaminase dans les lymphocytes T matures pour lesquels le traitement actuel est la transduction de lymphocytes T en culture prélevés par leucophérèse, par un vecteur rétroviral recombinant (pour une synthèse de ces essais voir Anderson W.F. Human Gene Therapy, Science (1992), 256 : 803-814).

Un autre type d'application du transfert de gènes dans les cellules hématopoïétiques est représenté par les infections virales graves (SIDA) ou la prévention de la stimulation immunitaire de type maladies auto-immunes ou GVHD (graft versus host disease). L'approche consiste à pièger les lymphocytes T en y incorporant un transgène constitué d'un gène suicide, par exemple la thymidine kinase de HSV1 (HSV1-TK) ou un équivalent fonctionnel de ce gène dont le produit d'expression peut métaboliser in situ une substance pharmaceutiquement inactive en un dérivé toxique pour induire spécifiquement la destruction de ces cellules, et dont un exemple dans le cas de l'HSV1-TK est le ganciclovir.

L'obtention de vecteurs d'expression ayant les qualités d'expression spécifiques des vecteurs de l'invention permet de surmonter les inconvénients cités plus haut des transferts dans les cellules souches ou dans les cellules matures.

Les vecteurs utilisables pour un transfert de gènes dans les cellules souches hématopoïétiques (CSH) et susceptibles d'incorporer les éléments de régulation de l'invention permettant l'expression spécifique du gène étranger exclusivement dans les cellules matures, peuvent être choisis parmi tous les vecteurs appropriés à la transfection de ces CSH. Il s'agit essentiellement des vecteurs viraux, de type adénovirus ou adéno associated virus (AAV), des vecteurs rétroviraux ou encore des systèmes non biologiques de délivrance in vivo d'acide nucléique. Une synthèse des caractéristiques essentielles de ces différents types de vecteurs pourra être trouvée dans : « Thérapie génique, Edition John Libbey, (1993) pages 3 à 33 ».

Des vecteurs rétroviraux particuliers ont été décrits pour pièger les cellules T soit à l'égard d'infection par les rétrovirus, soit pour induire une tolérance du GVHD, et décrits dans les demandes de brevets n° WO93/08843, WO93/08844, WO93/13824 et la demande de brevet français n° 9401994, dont les contenus sont incorporés par référence à la présente demande.

Dans le système d'expression de l'invention, dont le vecteur d'expression contient une cassette constituée du promoteur ou de la combinaison amplificateur/promoteur décrite plus haut, la protéine hétérologue susceptible d'être exprimée dans les lymphocytes T matures peut être choisie comme une protéine présentant une toxicité conditionnée par la présence d'une substance : la protéine hétérologue pouvant être avantageusement la thymidine kinase de HSV1-TK ou une protéine dérivée de celle-ci à partir du moment où elle conserve la caractéristique fonctionnelle, et un analogue de nucléosides tel l'aciclovir ou le ganciclovir susceptible d'être incorporé dans l'ADN en divisions après phosphorylation par la thymidine kinase et d'entraîner la mort du lymphocyte T mature.

Des exemples de constructions permettant la mise en oeuvre de cette approche « gène suicide » dans les lymphocytes T CD4+ par transfection d'un gène HSV1-TK et traitement par le ganciclovir ont été décrits dans Caruso M. et Klatzmann D. (Proc. Natl. Acad. Sciences (1992) 89 : 182-186).

La présente invention est également relative à des cellules de la lignée hématopoïétique notamment des CSH transfectées par un vecteur d'expression constitué au moins d'une séquence codant pour une protéine hétérologue, d'un promoteur et d'une séquence de polyadénylation, caractérisés en ce que ledit vecteur contient au moins une séquence amplificatrice d'un gène CD4 de mammifère de même espèce ou d'espèce différente, le cas échéant en combinaison avec un promoteur du gène CD4, la protéine hétérologue étant exprimée principalement dans les lymphocytes T matures issus de la sélection du répertoire.

De façon préférée le promoteur est constitué de l'une des séquences décrites plus haut constitué ou dérivé du promoteur du gène CD4 humain tel que représenté dans la figure 6.

Le promoteur peut également être un promoteur de cytokine ou d'un récepteur de cytokine comme celui de l'interleukine -2.

Dans les systèmes d'expression de l'invention, le vecteur d'expression contient, en amont ou en aval de la séquence à exprimer un amplificateur du CD4 murin, constitué de la séquence de 339 paires de bases décrites dans Sawada et al (1991). Un mode de réalisation est d'inclure entre 1 et 5 copies de cet amplificateur ou d'un amplificateur dérivé tel que défini plus haut et de préférence 3 copies.

Les cellules transfectées par un vecteur recombinant exprimant une protéine qui présente une toxicité conditionnelle pour les cellules T matures font également partie de l'invention ; notamment les cellules transfectées par un vecteur portant la thymidine kinase de HSV1-TK ou une protéine dérivée de celle-ci fait partie de l'invention. Le gène de la thymidine kinase peut être alors sous la dépendance d'un promoteur tel que décrit ci-dessus ou d'un promoteur spécifique, notamment le promoteur de l'interleukine -2, de toute façon combiné avec un amplificateur tel que décrit ci-dessus. Il doit être rappelé ici que pour les lymphocytes T, l'activation est associée à la division cellulaire ; puisque le ganciclovir n'est toxique que pour les cellules en division et exprimant HSV1-TK, il n'est donc pas nécessaire d'avoir un contrôle extrêmement strict de l'expression du gène HSV1-TK au cours de l'activation cellulaire. Une expression restreinte aux lymphocytes T matures est suffisante ; en effet, même si un lymphocyte T mature quiescent exprime de façon constitutive la protéine HSV1-TK, même en présence de ganciclovir, ce lymphocyte ne sera pas détruit. Au contraire dès que la cellule sera activée et se divisera, le ganciclovir triphosphate sera déjà présent dans la cellule et permettra d'éliminer celle-ci immédiatement.

La stratégie thérapeutique consistant à réaliser une greffe de moelle osseuse avec des cellules contenant un transgène HSV1-TK sous le contrôle des séquences régulatrices de l'invention présentera alors la sécurité requise de ne pas être exprimée pendant l'ensemble du processus de différenciation des lymphocytes T, et donc de ne pas perturber la sélection du répertoire, et de ne pas risquer de tuer des cellules en divisions permanentes que sont les thymocytes prématures. Enfin, et c'est le but recherché, le gène TK serait exprimé en permanence par tous les lymphocytes T. Dans le cas de la réinjection chez un patient de cellules souches de moelle osseuse, transduites avec TK, s'il apparaît une réaction du greffon contre l'hôte, qui implique l'activation de certains lymphocytes T du greffon reconnaissant des antigènes d'histocompatibilité du receveur, le système TK doit permettre l'élimination de ces lymphocytes grâce au traitement par le ganciclovir ou l'aciclovir.

La présente invention porte également sur l'utilisation de séquences régulatrices spécifiques du gène du CD4, notamment l'amplificateur, ou la combinaison de l'amplificateur et d'un promoteur tels que décrits plus haut, dans la fabrication d'un médicament utilisable en thérapie génique pour détruire spécifiquement les lymphocytes T activés, ces éléments de régulation étant intégrés dans un système d'expression susceptible de transformer les cellules du système hématopoïétique ; une telle utilisation permet d'envisager une expression spécifique du transgène incorporé dans le vecteur au niveau des cellules T matures CD4+ CD8- et CD4- CD8+, cette expression étant totalement réprimée pendant l'ensemble du processus de différenciation jusque et y compris la sélection du répertoire dans le thymus.

Nous avons rappelé plus haut que Sawada et al dans Cell (1991) ont mis en évidence l'existence d'un silenceur transcriptionnel spécifique du gène CD4 et qui est localisé dans le premier intron du gène. La combinaison du silenceur ainsi caractérisée avec les séquences régulatrices de l'invention, qu'elles soient amplificateur seul ou amplificateur combiné à un promoteur, amplificateur et promoteur gardant les définitions ci-dessus, font partie de l'invention ainsi que leur utilisation dans la fabrication d'un médicament utilisable en thérapie génique pour transformer les cellules souches du système hématopoïétique. Une telle combinaison d'éléments de régulation dans un vecteur d'expression d'une protéine hétérologue permet d'envisager l'expression de cette protéine hétérologue exclusivement dans les lymphocytes T matures CD4+ CD8- à l'exclusion des lymphocytes T immatures et des lymphocytes T cytotoxiques CD8+.

L'utilisation d'un système d'expression comprenant les vecteurs munis des séquences régulatrices de l'invention est particulièrement recommandée dans la fabrication d'un médicament de thérapie génique pour prévenir ou traiter les rejets de greffes, les réactions de du greffon contre l'hôte, ou bien encore pour soigner les maladies auto-immunes ; elle est également envisageable pour préparer un médicament utilisable pour prévenir et traiter les infections par des virus, et notamment par le VIH. L'utilisation de la construction comprenant en outre un silenceur peut permettre de préparer des médicaments pour la thérapie génique.

En effet, les données disponibles à ce jour laissaient penser que la combinaison amplificateur/promoteur/silenceur du gène CD4 devrait être suffisante pour obtenir une expression dans toutes les cellules CD4+ (y compris DP) Or, comme décrit dans cette invention, il apparaît que la combinaison amplificateur/promoteur a clairement un caractère innovant puisqu'elle entraîne une expression restreinte aux cellules T matures ; l'absence du silenceur n'intervient que dans l'expression dans les cellules CD8+ mais n'ést pas liée au stade de développement.

Les exemples ci-après permettrons d'illustrer les caractéristiques inattendues de la combinaison d'éléments régulateurs de l'invention et les propriétés qu'elle confère aux cellules dans lesquelles ils ont été intégrés.

L'homme du métier saura très facilement, à partir de la constatation décrite ici, adapter ensuite la construction qui devra être mise en oeuvre pour une prophylaxie ou une thérapeutique particulière qu'il voudrait mettre en oeuvre et nécessitant une expression spécifique dans les cellules T matures CD4+ CD8- et CD4- CD8+, ou uniquement dans les cellules T matures CD4+ CD8-.

Dans les exemples qui suivent les figures ont les légendes suivantes :
La figure 1A représente la cassette pCD4 constituée du promoteur du hCD4 de 1100 paires de bases, du cDNA du CD4 humain utilisé ici comme « reporter » et de la séquence de polyadénylation du virus SV40.
La figure 1B représente la cassette EpCD4 constituée de mêmes éléments que pCD4, pourvus en amont de 3 copies de la séquence de 339 paires de bases de l'amplificateur du CD4 de souris.
Dans les figures 1A et 1B, les lettres indiquent les sites de restriction avec les significations suivantes : H = Hind3, S = Sph1, P = Pst1, X = Xba1, B = BamH1, E = Eco R1.

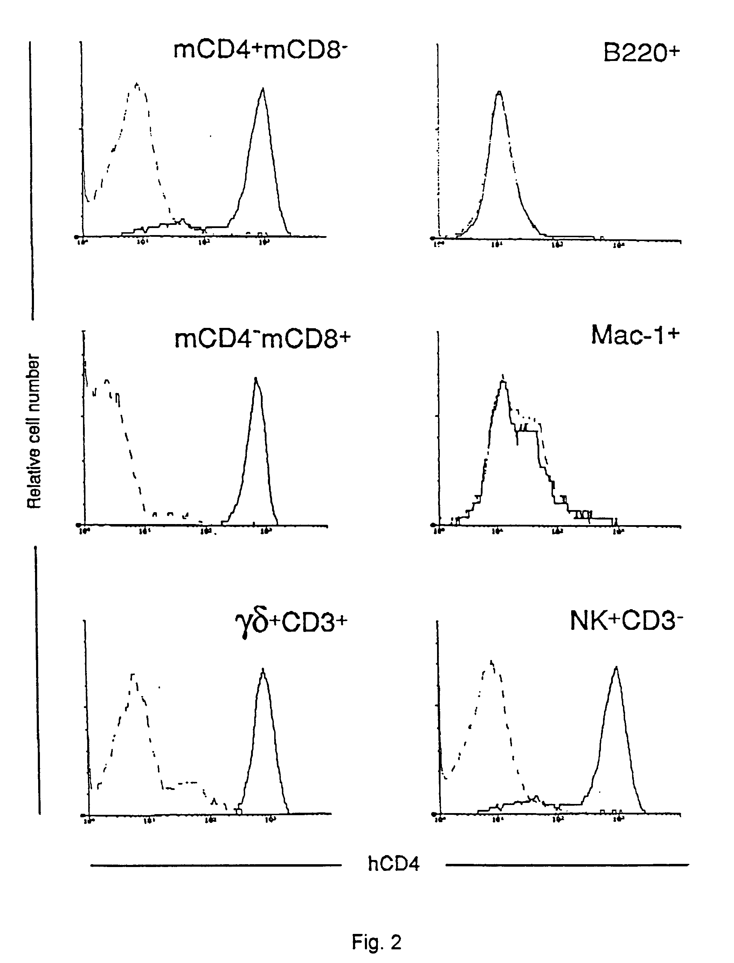

La figure 2 représente l'expression du gène reporter hCD4 à la surface des cellules mononuclées de la rate de souris transgèniques, respectivement les cellules SP CD4+, SP CD8+, lymphocytes γδ, lymphocytes B, macrophages et NK.

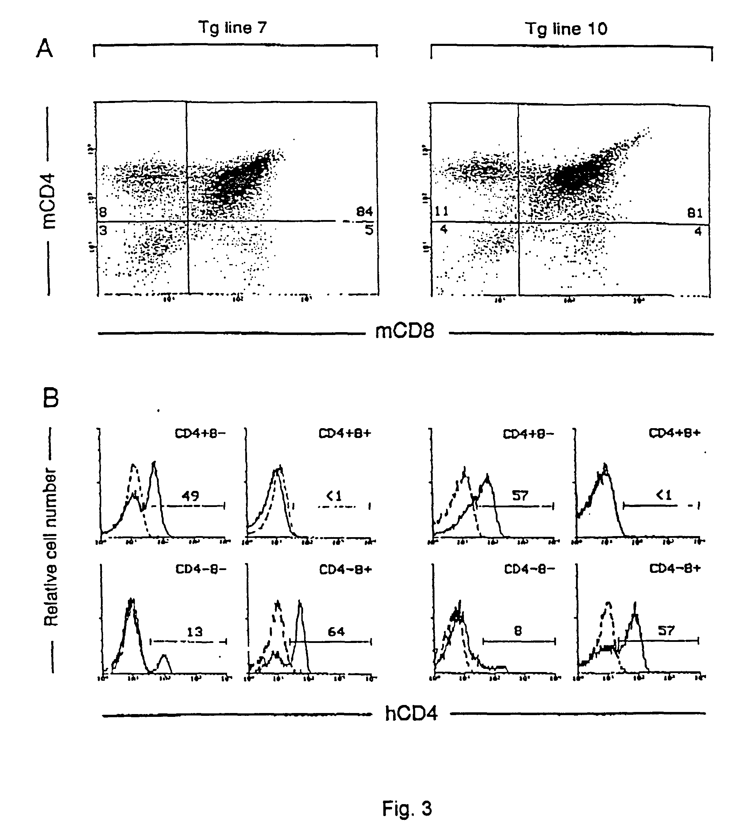

La figure 3 représente l'analyse de l'expression du gène reporter hCD4 dans les thymocytes de souris transgèniques par triple immuno-marquage et cytométrie de flux. (A) représente la distribution des sous-populations de thymocytes définies par l'expression de mCD4 et mCD8. Les nombres dans chaque cadran représentent le pourcentage de chaque sous-population dans le thymus. (B) représente l'expression de hCD4 dans les cellules DN (CD4- CD8-) ; DP (CD4+ CD8+) ; SP (CD4+ CD8-) et SP (CD4+ CD8+), respectivement.
   Les nombres représentent le pourcentage de thymocytes hCD4+ dans chaque sous-population pour les deux expériences présentes dans cette figure. Chaque résultat a été reproduit sept (lignée 7) et treize (lignée 10) fois lors d'expériences indépendantes.

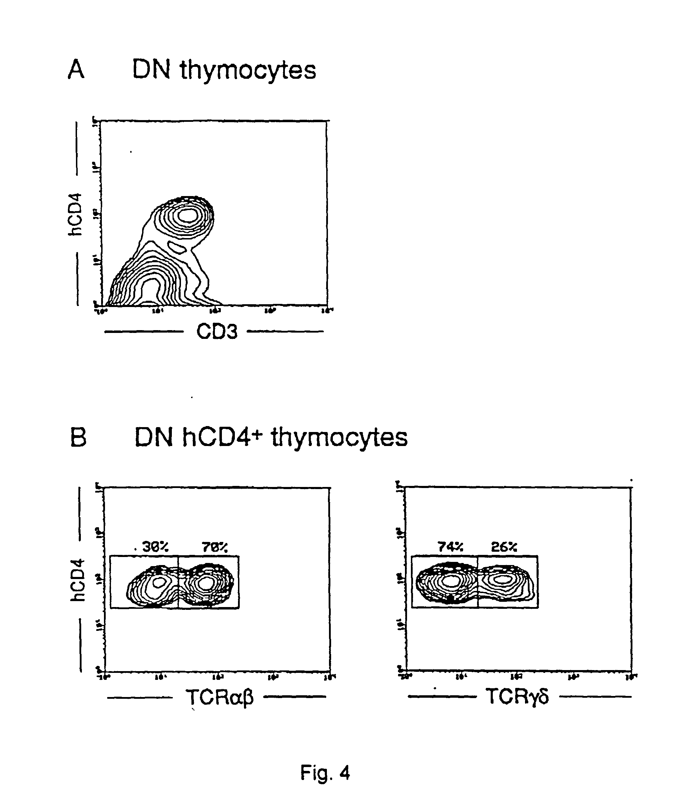

La figure 4 représente l'analyse de l'expression de CD3, TCR αβ et TCR γδ dans les thymocytes DN qui expriment hCD4. Les thymocytes de souris transgéniques ont été marqués par un anticorps monoclonal hCD4 couplé QR, un mélange d'anticorps mCD4 et mCD8 couplés PE et soit (A) un anticorps TCR αβ ou TCR γδ couplé FITC. Le phénotype (A) des thymocytes DN et (B) des thymocytes DN qui expriment hCD4 ont été étudiés en réalisant une fenêtre sur les cellules PE- et QR+PE-, respectivement. Les résultats présentés dans cette figure sont représentatifs de cinq (lignée 10) et une (lignée 7) expériences reproductibles.

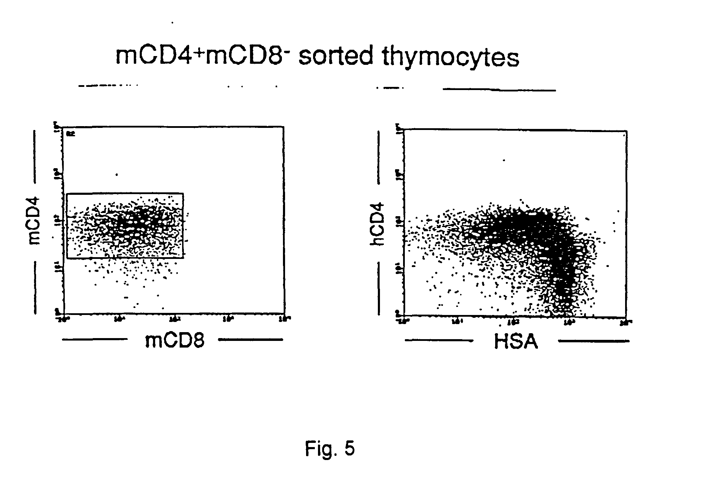

La figure 5 représente l'analyse de l'expression de hCD4 et HSA sur les thymocytes SP CD4. Les thymocytes SP CD4 ont été isolés par tri cellulaire, lavés, et ont subi un second immuno-marquage par les anticorps monoclonaux anti-HSA couplés PE et hCD4 couplés QR. L'expérience présentée a été reproduite trois (lignée 10) et une (lignée 7) fois lors d'expériences indépendantes.
La figure 6 représente la séquence du promoteur du gène codant pour le CD4 humain et extraite de P. Salmon et al. (Proc. Natl. Acad. Sci., USA 90 : 7739-7743 (1993)).

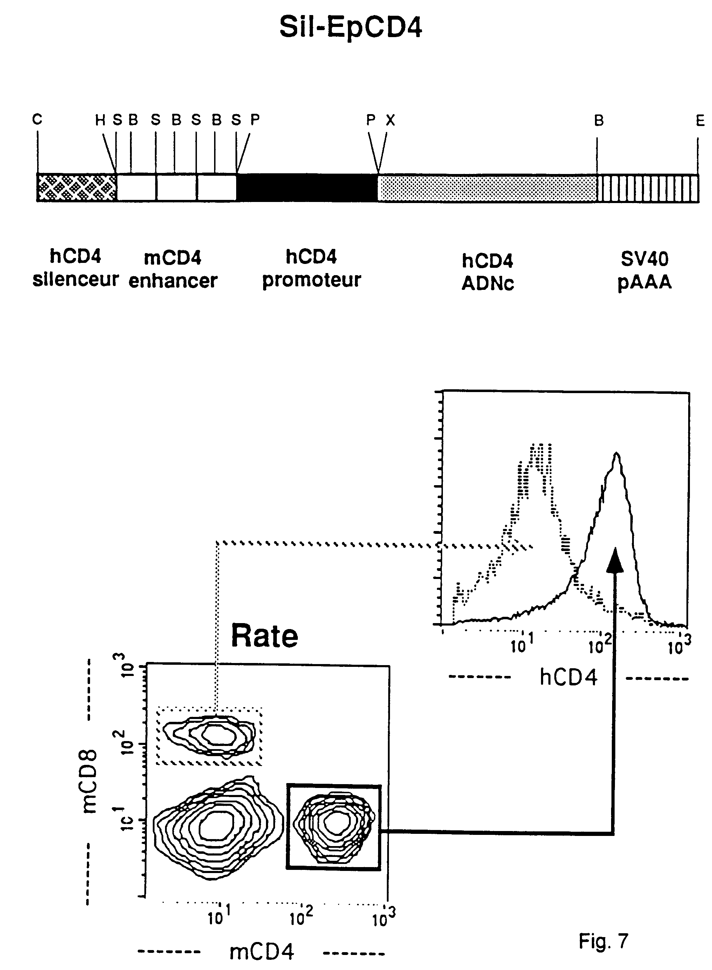

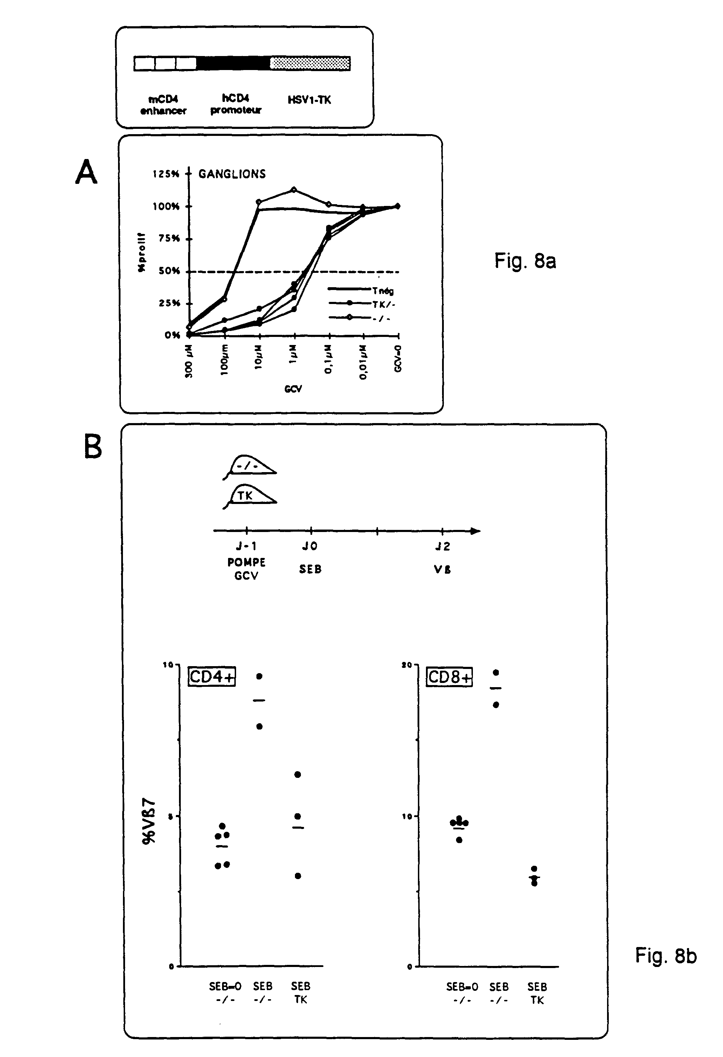

La figure 7 représente la caractérisation du silenceur du gène CD4 humain (Sil) permettant l'expression différentielle d'un transgène dans les lymphocites T CD4+ matures et non dans les lymphocytes CD8+ matures. La figure 8 représente la déplétion spécifique par le ganciclovir des lymphocytes T CD4+ et CD8+ (figure 8a) en réponse à un mitogène (ConA) in vitro et (figure 8b) la déplétion in vivo des lymphocytes T exprimant un TCR Vβ7 en réponse à une stimulation par le super-antigène SEB.

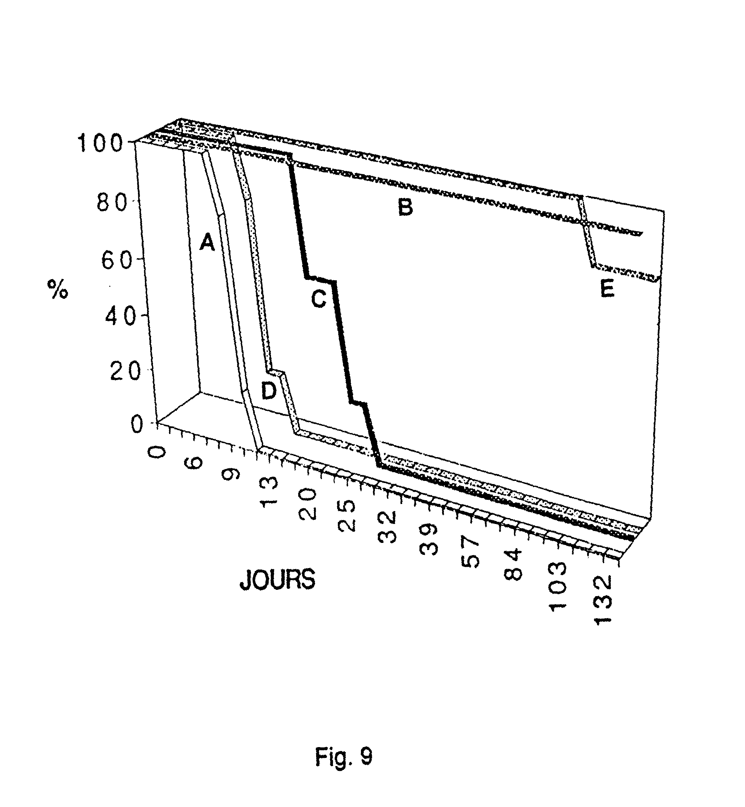

La figure 9 représente les résultats de l'analyse de souris irradiées qui reçoivent une greffe de moelle osseuse accompagnée ou non de splénocytes provenant, soit de souris transgéniques, soit de souris normales. Les animaux contrôles qui ne sont pas greffés (GMO-) meurent des conséquences de l'irradiation. Les animaux qui sont greffés avec une moelle osseuse seule sans splénocytes (GMO+) sont sauvés par la greffe de moelle osseuse, et il n'y a pas de GVH, comme cela est classique chez la souris. Les animaux recevant une greffe de moelle osseuse et des splénocytes allogéniques provenant, soit de souris transgéniques traitées avec le PBS (GVH/PBS), soit de souris non transgéniques traitées avec le ganciclovir (GVH/GCV NTG) meurent également rapidement et présentent tous les signes cliniques et histologiques d'une GVH. Seules les souris ayant reçu de la moelle osseuse et des splénocytes de souris transgéniques et traitées par le ganciclovir (GVH/GCV) survivent également à la greffe de moelle, sans signe histologique ni clinique de GVH.

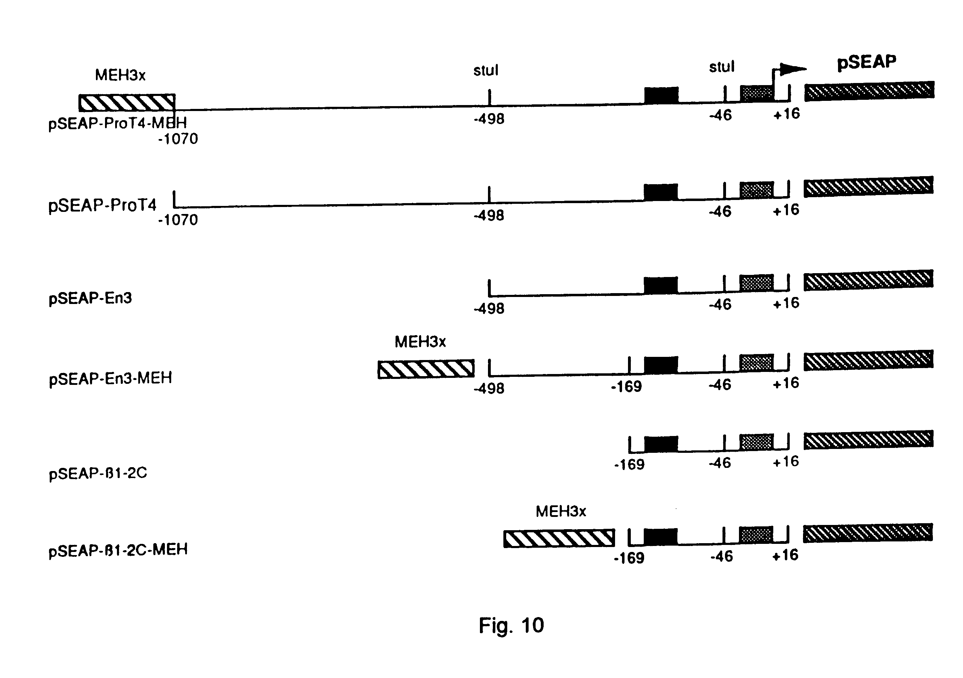

La figure 10 représente la caractérisation des séquences minimales permettant l'expression d'un transgène à partir des séquences régulatrices du gène CD4 humain.

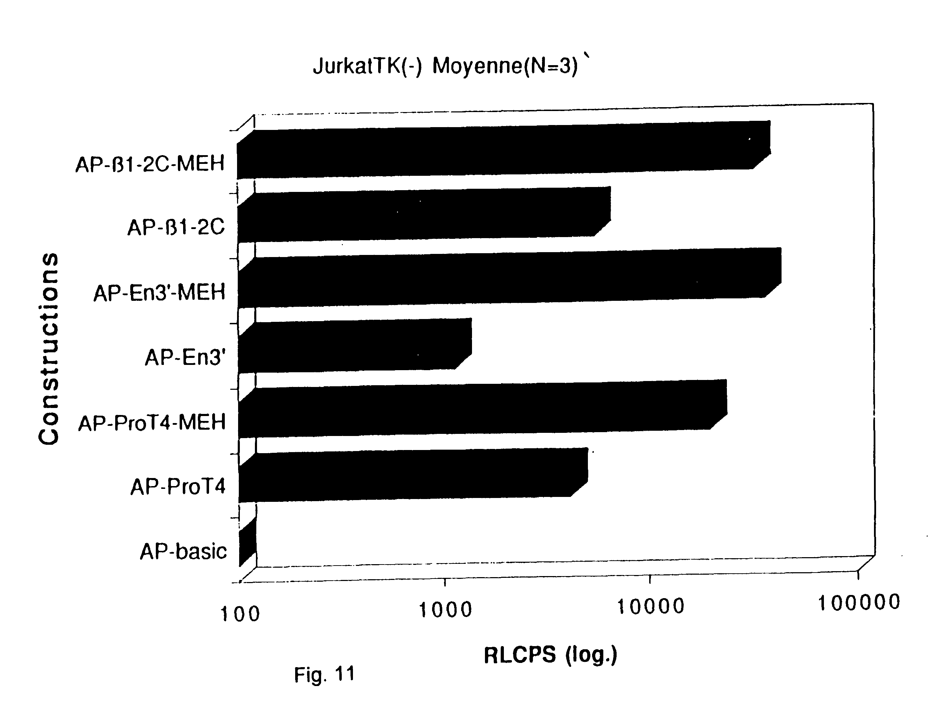

La figure 11 représente l'expression du gène rapporteur après transfection des différentes constructions génétiques de la ligéne 4 dans les cellules Jurkat.

### Exemple 1 : Expression d'un mini-gène CD4 humain dans les cellules T matures de souris transgèniques :

Deux plasmides ont été construits :
- le plasmide pCD4 portant une séquence de 1100 paires de bases correspondant au promoteur CD4 humain, avec en aval le cDNA du CD4 humain et une séquence de polyadénylation du SV40 (Figure 1A),
- le plasmide EpCD4 qui est la même construction comportant en outre trois répétitions de l'amplificateur CD4 murin de 339 paires de bases décrit dans Sawada (1991) (figure 1B).
   Le cDNA du CD4 appartient à ces constructions à titre de gène reporter c'est-à-dire dont on regarde l'expression ou la non expression dans le système de souris transgènique, l'expression étant facile à détecter par les techniques de cytométrie de flux utilisées.

### 1- Matériels et méthodes

### 1a- Construction des transgènes

Pour construire pCD4 représenté sur la figure 1A, le fragment Pst1 du promoteur humain CD4 (hCD4) décrit par P. Salmon et al, (1993), et comprenant les nucléotides -1076 à +20, a été ligaturé au fragment cDNA EcoR1-BamHI de 1,8 kb et codant pour la protéine CD4 humaine ; cette protéine a été caractérisée par Maddon P.J. et al dans Cell 42 : 93:104 (1985). La séquence de polyadénylation provient d'un fragment BamH1-BgI2 de 0,8 kb du virus SV40.

Dans la construction du plasmide EpCD4 (figure 1B) on a ajouté à la construction pCD4 un fragment amplifié par PCR de l'amplificateur CD4 murin directement en amont du promoteur hCD4 de la construction précédente. Brièvement, l'ADN génomique murin a été soumis à une amplification par PCR en utilisant des amorces complémentaires des séquences limitrophes de la séquence minimale de 339 paires de bases de l'amplificateur déjà décrit plus haut et contenant des sites de restriction additionnels Sph1. Les produits PCR ont été clônés dans le plasmide pCD4, et les séquences des inserts ont été vérifiées. Un clône contenant 3 copies de l'amplificateur murin a été retenu pour constuire les souris transgéniques.

### 1b- Génération et criblage des souris transgéniques

Des fragments Hind3-EcoRI de DNA ont été générés à partir des plasmides pCD4 et EpDC4, et séparés par électrophorèse en gel d'agarose. Les transgènes ont ensuite été élués en utilisant le système d'élution Biotrap (Schleicher et Schuell, Dassel, Germany) et ajustés à une concentration de 5 ng par µl dans 10 mM Tris-HCI, pH 7.4, EDTA 0.1 mM.

Les souris transgéniques porteuses des plasmides pCD4 et EpCD4 ont été obtenues et caractérisées comme décrits dans Hogan B et al (1986) dans Manipulating the Mouse Embryo, a Laboratory Manual ; Cold Spring Harbour Laboratory Press, Cold Spring Harbour, New York. Les transgènes ont été microinjectés dans les pronucléi des zygotes en F2 obtenus d'un croisement de deux : [C57 BI/6 DBA/2] F1. Les animaux transgéniques ont été détectés par hybridation du DNA préparé à partir de biopsies de queue, avec une sonde spécifique radiomarquée du CD4 humain qui est le fragment de 1,8 kb EcoRI-BamH1 du CD4 humain et décrit dans l'article de Maddon P. et al. (1985).

### 1c- Anticorps utilisés

Les anticorps monoclonaux directement marqués suivants ont été utilisés les sigles ayant les significations suivantes : FITC = isothiocyanate de fluorescéine, PE : phycoérythrine ; QR : quantum red ; SA : streptavidine; APC : allophycocyanine; PerCP : peridinine chrorophylle protéine; m : murin; h : humain.

Liste des anticorps :
anti-mCD4 (IgG2b de rat marquée PE ou APC, clône YTS 191.1),
anti-mCD8 (IgG2b de rat marquée FITC ou PE, clône YTS 169.4),
anti-mCD3 (IgG de hamster marquée FITC, clône 500-A2),
anti-mB220 (IgG2a de rat marquée FITC, clône RA3-6B2),
anti-mTCRαβ (IgG de hamster marquée FITC, clône H57-597) et
anti-mMAC1, (IgG2b de rat marquée FITC, clône M1-70.15),
provenant de Caltag Laboratories (San Franscisco, CA);
anti-mTCRγδ, (IgG de hamster marquée PE, clône GL3),
anti-mNK, (IgG2a de souris marquée PE, clônes PK 136 et 5E6),
anti-mHSA, (IgG2b de rat marquée PE, clône M1/69)
provenant de PharMingen (San Diego, CA);
anti-hCD4 (IgG1 de souris marquée QR, clône Q4120) et un contrôle négatif (IgG1 de souris marquée QR, MPOC-21) provenant de Sigma Immunochemicals (St Louis, MO)

### 1d- Préparation des cellules et analyse en cytométrie de flux

Toutes les analyses ont été réalisées sur des souris de 8 à 20 semaines. Le thymus, la rate et les ganglions lymphatiques sont dilacérés dans du DMEM (Dulbecco's modified Eagle medium) provenant de Life Technologies Inc. (Gaithersburg, MD), resuspendus à la pipette, centrifugés à 1100 TPM pendant 5 minutes, puis resuspendus dans un tampon de marquage composé de PBS contenant 2% de sérum de veau foetal et 0,02 % d'azide de sodium. La suspension de cellules spléniques a été mélangée à deux volumes de chlorure d'ammonium à 0,8% pour lyser les érythrocytes, puis centrifugée immédiatement et resuspendue dans le tampon de marquage. Après comptage, toutes les cellules en suspension sont ajustées à une concentration finale de 10 à 20 x10⁶ cellules/ml. Pour les analyses des cellules B et des macrophages, une concentration finale de 2% de sérum autologue a été ajoutée au tampon de coloration avant l'addition des anticorps monoclonaux. Les marquages ont été réalisés en incubant 1 à 2x10⁶ cellules avec les anticorps monoclonaux pendant 30 minutes à 4°C ; Après un lavage final, les cellules ont été fixées dans 0,5 à 1 ml de 1% de paraformaldehyde dans du PBS. 10 à 40 000 cellules ont ainsi été analysées en cytométrie de flux (FACScan, Becton Dickinson, San Jose, CA).

Des thymocytes SP CD4+ CD8- purifiés ont été obtenus après double marquage coloration d'une suspension cellulaire thymique totale avec des anticorps anti-mCD4 marqués à l'APC et des anticorps mCD8 marqués PE, pendant 1 heure dans la glace, puis lavés et filtrés à travers un filtre cellulaire en nylon de type Falcon 2350 (Becton Dickinson, Franklin Lakes, NJ). Les cellules APC+ et PE- sont triées avec un FACStarPlus (Becton Dickinson) dans du tampon PBS avec 0,02% d'azide de sodium.

Lorsque 10⁶ cellules SP CD4+ CD8- ont été obtenues par tri cellulaire, elles sont lavées et resuspendues dans 250 µl de tampon de marquage, et séparées en deux tubes et soumises à un marquage avec des anticorps anti-HSA marqués à la PE, et soit des anti-hCD4 marqués QR ou des anticorps isotypiques contrôles. Les cellules ont été analysées sur un FACScan et analysés comme décrit ci-dessus.

### 2- Résultats

### 2-1 Une expression détectable du gène reporter constitué du cDNA du CD4 in vivo nécessite la présence de l'amplificateur en sus du promoteur du CD4.

Le tableau 1 ci-dessous résume le pourcentage des cellules exprimant le gène reporter hCD4 au niveau du thymus, de la rate, des ganglions lymphatiques et des CMNSP (cellules mononuclées du sang périphérique), la présence du CD4 a été analysée par cytométrie de flux comme décrit ci-dessus. Les chiffres entre parenthèses indiquent le nombre d'animaux testés ; et les résultats indiquent la moyenne obtenue entre les différents animaux testés plus ou moins l'écart type.

**TABLEAU 1**

| Construction | Thymus | Rate | Ganglions Lymphatiques | CMNSP |
|---|---|---|---|---|
| pCD4 (7 lignées) | <1 | <1 | N.D. | <1 |
| EpCD4 | 8±3.0 (10) | 30±6.1 (10) | 75±13.4 (6) | 58±14.0 (6) |

Sept lignées transgéniques ont été obtenues portant 1 à 40 copies du plasmide pCD4, ne comportant donc pas l'amplificateur ; aucune cellule hCD4 positive n'a pu être détectée dans ces conditions. Pour la construction EpCD4, quatre lignées ont été obtenues avec 5 à 20 copies de la construction qui expriment toutes le transgène hCD4, deux ont été analysés en détail (lignée 7 et lignée 10). Les résultats du tableau pour EpCD4 ont été obtenus dans la lignée 10 et des résultats similaires ont été obtenus dans la lignée 7. Les cellules de la rate, des ganglions lymphatiques et des CMNSP sont des cellules matures obtenues après la sélection du répertoire ce qui explique le pourcentage nettement plus important des cellules exprimant le CD4 dans ces cellules que celui obtenu dans le thymus. En conclusion, il apparaît que la présence de l'amplificateur du gène CD4 est nécessaire et suffisante pour obtenir l'expression du gène reporter.

### 2.2- Expression du transgène EpCD4 dans les organes lymphoïdes périphériques.

Les lignées transgèniques 10 et 7 de EpCD4 ont été analysées pour connaître les types cellulaires qui expriment le transgène. Ainsi, nous avons réalisé des analyses en cytométrie de flux après double ou triple marquages sur des splénocytes en utilisant des anticorps monoclonaux contre le CD4 humain en combinaison avec des anticorps monoclonaux reconnaissant différents types cellulaires hématopoïétiques.

Comme le montre la figure 2, l'expression de hCD4 est restreinte aux lymphocytes T et aux cellules NK. L'expression de hCD4 a été détectée dans toutes les cellules T au même niveau, que ces dernières expriment CD4 ou CD8. L'expression est comparable dans les cellules lymphocytes exprimant un récepteur γδ. Aucune expression n'a pu être détectée ni dans les monocytes ni dans les lymphocytes B des souris analysées (figure 2D-E).

La majorité des cellules NK, définies comme étant reconnues tant par les anticorps monoclonaux PK136 que 5E6, ont également été trouvées comme exprimant le hCD4 en proportion variable (figure 2F). L'ensemble de ces résultats indique que l'expression du transgène EpCD4 dans les cellules périphériques est restreinte aux cellules T et aux cellules NK.

Les résultats de cette figure ont été reproduits trois à douze fois lors d'expériences indépendantes. Lors de l'analyse des cellules NK, deux anticorps monoclonaux différents ont été utilisé (PK136 et 5E6) révélant une proportion de cellules hCD4+ allant de 74 à 100%. Ces fréquences sont toujours légèrement plus élévées dans les cellules 5E6+ que dans les cellules PK136+.

Les résultats obtenus pour les lymphocytes SP CD4+ et SP CD8+ ont été reproduits pour les ganglions lymphatiques et les cellules mononuclées du sang périphérique.

Ces résultats ont été obtenus dans la lignée 10, et des résultats comparables ont été obtenus dans la lignée 7.

### 3- Expression du transgène EpCD4 dans les thymocytes.

Dans la figure 3 qui représente l'analyse de l'expression du gène reporter hCD4, la fréquence moyenne de thymocytes hCD4+ trouvée dans les DN, DP, SP CD4 et SP CD8 sont (moyenne plus ou moins écart-type) : 21% plus ou moins 6,7%, inférieur à 1%, 62% plus ou moins 8,4% et 67% plus ou moins 18,1% pour la lignée 10 (n=13), et 13% plus ou moins 6,0%, inférieur à 1%, 41% plus ou moins 12,9% et 68% plus ou moins 13,0% pour la lignée 7 (n=7). Le hCD4 est indétectable dans les thymocytes DP alors qu'une fraction très significative des SP exprime le transgène.

L'expression du hCD4 a été détectée dans une fraction minoritaire des thymocytes DN (10 à 25% en moyenne). Bien que ces cellules DN et hCD4+ représentent moins de 1% des thymocytes totaux, il était important de vérifier leur phénotype pour savoir s'il s'agit de précurseurs thymiques précoces DN ou des cellules T matures DN (exprimant alors un TCR/CD3). Pour cela il a été réalisé un triple marquage avec :
- un mélange d'anticorps anti-CD4 et anti-CD8 marqués PE,
- un anticorps anti-hCD4 marqué QR,
- un anticorps soit anti-CD3, soit anti-TCR αβ, soit anti-TCR γδ, marqués FITC.

Comme le montre la figure 4, les thymocytes doubles négatifs et hCD4+ sont tous constitués de cellules exprimant un récepteur CD3/TCR ; parmi ces cellules 3/4 expriment le TCR αβ alors que 1/4 expriment le TCR γδ. Cela signifie donc que le transgène EpCD4 est exprimé non pas dans des thymocytes immatures DN mais dans des thymocytes matures DN qui expriment un récepteur pour l'antigène.

Au contraire le transgène EpCD4 a été trouvé être exprimé dans une fraction très importante des thymocytes SP quelque soit leur phénotype CD4+ ou CD8+ (figure 3).

Ces résultats sont très surprenants et ne pouvaient être prévus d'après les données disponibles à ce jour.

Il ressort de ce qui précède que le mini-gène CD4 est exprimé dans toutes les cellules T périphériques et seulement sur une fraction des thymocytes SP ; la question se pose donc de savoir si cette expression dans les SP est corrélée au étapes de différenciation des thymocytes SP.

Pour répondre à cette question des thymocytes SP CD4+ CD8-ont été analysés quant à l'expression de HSA et hCD4 à leur surface. Ceci est représenté dans la figure 5 et indique clairement que seuls les thymocytes les plus matures (HSA-) expriment hCD4 alors que leurs précurseurs HSA+ ne l'expriment pas et qu'il existe donc une filiation hCD4- HSA+ vers hCD4+ HSA- dans les SP CD4+.

Ces résultats montrent que l'expression du transgène EpCD4 est directement liée à l'étape de maturation des cellules T quelle que soit la lignée CD4+ ou CD8+ : elle apparaît sur les thymocytes SP les plus matures puis se maintient en périphérie.

Au total, cette combinaison de séquences régulatrices (promoteur+amplificateur) permet de contrôler l'expression d'un gène reporter dans les seules cellules T matures et une proportion variable de cellules NK. Il s'agit d'une observation qui n'était pas prévisible car les données disponibles jusqu'à ce jour suggéraient que la combinaison de ces éléments devait gouverner l'expression d'un gène reporter dès le stade DP. Ils impliquent l'existence d'un élément régulateur additionnel non encore identifié permettant d'obtenir l'expression dans les DP et qui pourrait être présent dans le premier intron du gène CD4.

### Exemple 2 : Expression d 'un mini-gène HSV1-TK dans les cellules T matures de souris transgéniques :

Un Plasmide EpTK a été construit ; il est composé des mêmes éléments régulateurs que EpCD4 précédemment décrit, soit 3 copies de l'activateur (enhancer) murin, le promoteur CD4 humain proT4 et le signal PolyA de SV40. Il diffère de EpCD4 en ce que le cDNA de hCD4 a été remplacé par un fragment de 1,3 kb contenant l'ADN codant pour HSV1-TK. Des souris transgéniques ont été réalisées pour détruire spécifiquement grâce à un traitement par le ganciclovir les lymphocytes T matures CD4+ et CD8+ en division (activés). Cette destruction touche également les DN CD3+, les lymphocytes γδ. et une proportion de cellules NK. En revanche, cette destruction ne touche pas les précurseurs thymocytaires immatures DP qui représentent la principale population thymique. Les lymphocytes T provenant de ganglions lymphatiques des souris transgéniques soient détruits in vitro par le ganciclovir, lorsqu'ils sont cultivés en présence d'un mitogène comme la concanavaline A.

Dans ces souris transgéniques, utilisées pour étudier l'homéostasie et le taux de renouvellement des lymphocytes T, une disparition progressive d'une proportion de lymphocytes T matures au cours du temps sous traitement par le ganciclovir est observée.

Quand ces souris transgéniques subissent une délétion provoquée des lymphocytes répondeurs à un antigène donné, une tolérance spécifique d'antigène lorsque le ganciclovir est administré dans la période entourant l'immunisation par l'antigène est obtenue.

Dans ces souris transgéniques, on observe un contrôle d'une réaction du greffon contre l'hôte lors du traitement par le ganciclovir, quand cette réaction a été créée par greffe de moelle osseuse mélangée à des lymphocytes T matures provenant de souris EpTK. Un autre résultat en est le traitement ou la prévention de la réaction du greffon contre l'hôte avec maintien de la réaction du greffon contre la leucémie (GVL, graft versus leukemia).

Dans des souris transgéniques réalisées avec des constructions de type EpTK modifiées en ajoutant divers éléments régulateurs précédemment cités provenant du gène CD4 humain ou d'autres espèces, et en particulier le silenceur du gène CD4 dans les lymphocytes CD8 et diverses séquences provenant du premier intron du gène CD4, des populations T où s'exprime HSV1-TK lorsque celles-ci sont en division, sont détruites par le ganciclovir.

Différentes constructions contenant HSV1-TK et placées sous le contrôle des séquences régulatrices précédemment décrites provenant du gène CD4 de différentes espèces animales peuvent être réalisées dans le but de fabriquer des vecteurs d'expression. Ces vecteurs sont d'une part des vecteurs non viraux, et d'autre part des vecteurs viraux, notamment rétrorivaux ou dérivés d'AAV ou d'adénovirus. Tous ces différents vecteurs peuvent être utilisés pour transduire le gène HSV1-TK sous contrôle des différentes séquences régulatrices précédemment décrites dans, soit les lymphocytes T périphériques cultivés ex vivo, soit les cellules de moelle osseuse et notamment les cellules souches hématopoïétiques. Un résultat attendu est la reproduction de la spécificité d'expression obtenue avec les souris transgéniques exprimant HSV1-TK sous contrôle des mêmes séquences régulatrices que celles du vecteur, soit après sa transduction dans les lymphocytes T périphériques ex vivo, soit après sa transduction dans les cellules souches hématopoïétiques.

### Exemple 3 : Construction génétique permettant une expression spécifique dans les lymphocytes T CD4+ matures :

L'addition à la construction, contenant le promoteur et l'enhancer de CD4, d'une séquence dite « silencer de CD4 » déjà identifiée, permet la réalisation d'une construction génétique dont l'expression est maintenant restreinte exclusivement aux lymphocytes TCD4+ matures chez les souris transgéniques (figure 7). Les thymocytes doubles négatifs, les thymocytes doubles positifs, et les lymphocytes TCD8+ matures, n'expriment pas le transgène. En conséquence, ce type de construction peut être utilisé pour véhiculer un transgène de façon exclusive dans les T CD4+ matures, par exemple dans une perspective de thérapie génique, lorsque ces séquences régulatrices sont incluses dans un vecteur rétroviral.

### - délétion de clones T spécifiques :

Les séquences régulatrices promoteurs de CD4 et enhancer de CD4 préalablement utilisées chez la souris transgénique pour une expression exclusivement dans les lymphocytes T CD4+ ou CD8+ matures, ont été utilisées pour l'expression d'un gène suicide HSV1 TK. Des souris transgéniques ont été réalisées avec cette construction génétique.

La fonctionnalité de la construction génétique est démontrée par culture de lymphocytes matures des ganglions de ces souris transgéniques en présence de ganciclovir, après activation in vitro par un mitogène. Les concentrations de ganciclovir toxiques pour les cellules de souris transgéniques sont cent fois plus faibles que celles nécessaires pour tuer des lymphocytes T de souris non transgéniques (figure 8a).

Cette fonctionnalité ayant été démontrée, la démonstration de l'utilité de l'expression d'un gène HSV1 TK sous le contrôle de ces séquences régulatrices pour des applications thérapeutiques, peut être analysée. L'idée générale est de détruire les cellules lorsqu'elles sont activées par l'antigène dans des situations où des lymphocytes T sont responsables de pathologies. La démonstration de la faisabilité de ce procédé peut être réalisée en injectant chez les souris transgéniques et les souris contrôles un super antigène connu pour activer spécifiquement les lymphocytes porteurs d'un Vβ7. Dans les souris contrôles, que ce soit dans les populations CD4+ ou CD8+, l'activation par le super antigène SEB aboutit à un doublement du pourcentage des cellules Vβ7. Au contraire, en présence du traitement par le ganciclovir, il n'y a pas de modification du nombre de ces cellules (figure 8b). Ces résultats montrent qu'il est possible d'obtenir une destruction spécifique de cellules activées par un antigène lorsque les lymphocytes T expriment le gène HSV1 TK sous contrôle de ces séquences régulatrices.

### - traitement de la réaction du greffon contre l'hôte :

La fonctionnalité de ce système pour le contrôle d'une réaction du greffon contre l'hôte a été testée. Des souris irradiées sont reconstituées avec des cellules de moelle osseuse et des splénocytes provenant de souris transgéniques exprimant le gène HSV1-TK sous le contrôle du promoteur de CD4 et des enhancers de CD4. Cette greffe de moelle est réalisée dans un contexte allogénique, et dans cette situation, les splénocytes réinjectés en même temps que la moelle osseuse sont responsables d'une réaction du greffon contre l'hôte mortel.

Dans ces expériences, on observe 100 % de mortalité chez les animaux ayant reçu une telle greffe de moelle en condition allogénique. Au contraire, le traitement par le ganciclovir pendant les sept jours qui suivent la greffe de moelle est suffisant pour empêcher totalement le développement d'une réaction du greffon contre l'hôte. Dans ces conditions, la plupart des animaux survivent à la greffe de moelle, sans signe de GVH (figure 9).

Le tableau ci-dessous montre le pourcentage de survie avec les souris transgéniques TK à partir des résultats de 3 expériences allant de J 120 à J 41 effectuées sur des souris irradiées :

**TABLEAU 2:**

| GROUPES | GMO+ | CONTROLES | TRAITES |
|---|---|---|---|
| | 84% (n = 13) | 0% (n = 23) | 93% (n = 14 |

Le tableau ci-dessus fait apparaître que les souris irradiées recevant une greffe de moelle osseuse (GMO+) survivent, tandis que les mêmes souris recevant de la moelle osseuse et des splénocytes meurent de GVH (contrôle) sauf lorsque les splénocytes proviennent de souris transgéniques et que les souris sont traitées par le ganciclovir (traitées).

La démonstration que ce phénomène est bien due à une destruction des clones de lymphocytes T impliqués dans la GVH, peut être apportée par l'étude de la fonctionnalité des lymphocytes de ces souris. En effet, lorsque l'on prélève ces lymphocytes et qu'on les active dans des réactions mixtes lymphocytaires utilisant comme cellules stimulantes, soit des lymphocytes de même origine que le receveur, soit des lymphocytes d'une tierce origine, on constate qu'il n'y a aucune activation contre les cellules du receveur, tandis qu'il y a une activation normale contre les tierces cellules. Ces résultats démontrent qu'on a abouti, chez les souris ayant survécu à la greffe de moelle, à une délétion des clones spécifiquement capables de reconnaître les allo-antigènes du receveur, et impliqués dans la GVH.

### Exemple 4 : Constructions contenant différentes séquences du promoteur CD4 humain :

Différentes constructions génétiques ont été réalisées afin de rechercher quelles sont les séquences régulatrices minimales dérivées des séquences du gène CD4 qui permettent l'expression d'un transgène spécifiquement dans les lymphocytes T. Toutes ces constructions sont utilisées pour contrôler l'expression d'un gène rapporteur P. La construction de base contient les 1100 paires de base du promoteur du gène CD4 et l'enhancer de CD4 présent en 3 exemplaires. A partir de cette construction, différentes autres constructions génétiques ont été réalisées selon le schéma de la figure 10.

Les résultats de l'analyse de l'expression du gène rapporteur après transfection de ces différentes constructions génétiques de la lignée 4 dans les cellules Jurkat montrent (figure 11) que :
1) de façon générale l'expression est 10 fois plus élevée en présence de l'enhancer du CD4 qu'en son absence ;
2) le fragment -169+ 16 de la construction Pβ1-2C est aussi efficace que l'ensemble du fragment de 1100 paires de base pour diriger l'expression du gène rapporteur.

En conclusion, ces résultats montrent que les séquences régulatrices courtes dérivées du promoteur et enhancer du gène CD4 et de taille compatible avec leur utilisation au sein de vecteurs viraux tels les vecteurs rétroviraux sont utilisables pour obtenir une expression spécifique dans les lymphocytes T.

Les mêmes constructions dérivées des séquences régulatrices de CD4 ne s'expriment pas dans des cellules non lymphoïdes CD4+ comme, par exemple, les cellules HELA.

Ces expériences montrent également que la présence d'une seule copie de l'enhancer CD4 est suffisante pour obtenir l'expression attendue.

Ci-après se trouvent les légendes complémentaires des figures 9 et 10.

Figure 9 : le groupe GMO- est réprésenté par la courbe A. Le groupe GMO+ est représenté par la courbe B. Le groupe GVH/GCV NTG est représenté par la courbe C. Le groupe GVH/PBS est représenté par la courbe D et le groupe GVH/GCV est représenté par la courbe E.

Figure 10 : Le site d'initiation de la transcription du gène CD4 humain est représenté par une flèche noire. Le gène de la phosphatase alcaline sécrétée humaine (rapporteur) est, quant à lui, matérialisé par le sigle pSEAP. Les rectangles noirs et grisés représentent les séquences homologues avec la souris respectivement 2 « boîtes » conservées et 3 « boîtes » conservées. Enfin, « MEH3X » correspond au enhancer du gène CD4 murin qui, dans le présent test, est répété trois fois.

## Revendications

1. Système d'expression d'une protéine ou d'un gène hétérologue comprenant un vecteur recombinant, utilisable pour la transfection de cellules de la lignée hématopoïétique, notamment les cellules souches, de sorte que ladite cellule ainsi transduite n'exprimera la protéine ou le gène hétérologue que dans les cellules T matures après la sélection du répertoire, ledit vecteur étant pourvu de toutes les séquences nécessaires à son expression, et **caractérisé en ce qu'**il contient au moins un amplificateur d'un gène CD4 de même espèce ou d'espèce différente.

2. Système d'expression selon la revendication 1 **caractérisé en ce que** le vecteur comprend également un promoteur constitué de l'une des séquences suivantes :
- le promoteur d'un gène CD4, notamment le promoteur du gène CD4 humain tel que représenté dans la figure 6, ou
- la séquence comprise entre les nucléotides 579 et 1092 de la figure 6, ou
- la séquence comprise entre les nucléotides 912 et 1092 de la figure 6
- toute séquence dérivée de l'une des séquences précédentes par addition, délétion ou substitution de nucléotides sans modification substantielle de l'expression de la protéine hétérologue.

3. Système d'expression selon la revendication 1 ou 2 **caractérisé en ce que** la séquence amplificatrice est l'amplificateur du CD4 murin et comprend tout ou partie de la séquence suivante sous forme d'une à plusieurs copies :

TGTTGGGGTT CAAATTTGAG CCCCAGCTGT TAGCCCTCTG CAAAGAAAAA
AAAAAAAAAA AAAGAACAAA GGGCCTAGAT TTCCCTTCTG AGCCCCACCC
TAAGATGAAG CCTCTTCTTT CAAGGGAGTG GGGTTGGGGT GGAGGCGGAT
CCTGTCAGCT TTGCTCTCTC TGTGGCTGGC AGTTTCTCCA AAGGGTAACA
GGTGTCAGCT GGCTGAGCCT AGGCTGAACC CTGAGACATG CTACCTCTGT
CTTCTCATGG CTGGAGGCAG CCTTTGTAAG TCACAGAAAG TAGCTGAGGG
GCTCTGGAAA AAAGACAGCC AGGGTGGAGG TAGATTGGT

4. Système d'expression selon l'une des revendications 1 à 3 **caractérisé en ce que** le vecteur contient en outre, sous la dépendance du promoteur CD4 ou l'un de ses dérivés, tout ou partie du cDNA du gène CD4 humain.

5. Système d'expression selon l'une des revendications 1 à 4 **caractérisé en ce que** le vecteur contient en outre un silenceur de l'expression du gène CD8.

6. Système d'expression selon l'une des revendications 1 à 5 **caractérisé en ce que** la protéine hétérologue susceptible d'être exprimée dans les lymphocytes T matures, présente une toxicité conditionnelle pour le lymphocyte T mature.

7. Système d'expression selon la revendication 6 **caractérisé en ce que** la protéine hétérologue est la thymidine kinase de HSV1-TK ou une protéine dérivée de celle-ci, susceptible d'entraîner la mort du lymphocyte T activé en présence d'un analogue de nucléotide tel l'aciclovir ou le ganciclovir.

8. Système d'expression selon l'une des revendications 1 à 7 **caractérisé en ce que** le vecteur est soit :
- un vecteur rétroviral, et l'amplificateur et le cas échéant le promoteur issu du promoteur CD4 humain étant insérés entre les deux séquences LTR du vecteur rétroviral,
- un vecteur adénoviral, ou un vecteur AAV, ou
- un système non biologique de délivrance d'acides nucléiques.

9. Cellule de la lignée hématopoïétique transfectée par un vecteur d'expression constitué au moins d'une séquence codant pour une protéine hétérologue, d'un promoteur et d'une séquence de polyadénylation, **caractérisée en ce que** ledit vecteur contient au moins une séquence amplificatrice d'un gène CD4 de mammifère de même espèce ou d'espèce différente, la protéine hétérologue étant exprimée principalement dans les lymphocytes T matures issus de la sélection du répertoire.

10. Cellule transfectée selon la revendication 9 **caractérisée en ce que** le vecteur contient un promoteur constitué de l'une des séquences suivantes :
- le promoteur d'un gène CD4, notamment le promoteur du gène CD4 humain tel que représenté dans la figure 6, ou
- la séquence comprise entre les nucléotides 579 et 1092 dans la numérotation de la figure 6, ou
- la séquence comprise entre les nucléotides 912 et 1092 dans la numérotation de la figure 6, ou
- toute séquence dérivée de l'une des séquences précédentes par addition, délétion ou substitution de nucléotides sans modification substantielle de l'expression de la protéine hétérologue.

11. Cellule transfectée selon l'une des revendications 9 à 10 **caractérisée en ce que** la protéine hétérologue présente une toxicité conditionnelle pour les cellules T matures.

12. Cellule transfectée selon l'une des revendications 9 à 11 caractérisée en ce la protéine hétérologue est la thymidine kinase de HSV1-TK ou une protéine dérivée de celle-ci, susceptible d'entraîner la mort du lymphocyte T activé en présence d'un analogue de nucléotide tel l'aciclovir ou le ganciclovir.

13. Utilisation d'un système d'expression selon l'une des revendications 1 à 8 pour la fabrication d'un médicament utilisable en thérapie génique pour détruire spécifiquement les lymphocytes T activés.

14. Utilisation selon la revendication 13 pour la fabrication d'un médicament destiné à prévenir le rejet de greffe ou la réaction du greffon contre l'hôte.

15. Utilisation selon la revendication 13 pour la fabrication d'un médicament destiné à prévenir ou traiter des infections virales et notamment par le VIH.

16. Utilisation selon la revendication 13 pour la fabrication d'un médicament destiné à prévenir ou traiter les maladies auto-immunes.

17. Utilisation selon la revendication 13 pour la fabrication d'un médicament destiné à prévenir ou traiter les déficits immunitaires primitifs ou secondaires.

18. Composition thérapeutique destinée à prévenir le rejet de greffe **caractérisée en ce qu'**elle contient un système d'expression d'un transgène selon l'une des revendications 1 à 8.

19. Composition thérapeutique destinée à prévenir ou traiter des infections virales, notamment par le VIH, **caractérisée en ce qu'**elle contient un système d'expression d'un transgène selon l'une des revendications 1 à 8.

20. Composition thérapeutique destinée à prévenir ou traiter les maladies auto-immunes **caractérisée en ce qu'**elle contient un système d'expression d'un transgène selon l'une des revendications 1 à 8.

21. Composition thérapeutique destinée à prévenir ou traiter la réaction du greffon contre l'hôte **caractérisée en ce qu'**elle contient un système d'expression d'un transgène selon l'une des revendications 1 à 8.

22. Utilisation d'une séquence amplificatrice d'un gène CD4, notamment humain, pour la préparation d'un médicament permettant l'expression spécifique d'un transgène dans les lymphocytes T matures.

23. Utilisation selon la revendication 22, d'une séquence amplificatrice et d'un promoteur d'un gène CD4.

## Patentansprüche

1. Expressionssystem eines Proteins oder eines heterologen Gens, das einen rekombinanten Vektor umfasst, der zur Transfektion von Zellen der hämapoietischen Linie, insbesondere von Stammzellen, verwendet werden kann, derart dass die transfizierte Zelle das Protein oder das heterologe Gen nur in reifen T-Zellen nach der Auswahl des Repertoires exprimiert, wobei der Vektor mit allen zu seiner Expression nötigen Sequenzen versehen ist und **dadurch gekennzeichnet** ist, dass er mindestens einen Enhancer eines CD4-Gens der gleichen oder einer anderen Spezies enthält.

2. Expressionssystem nach Anspruch 1, **dadurch gekennzeichnet**, dass der Vektor auch einen Promotor umfasst, der aus einer der folgenden Sequenzen besteht:
- aus dem Promotor eines CD4-Gens, insbesondere aus dem Promotor des humanen CD4-Gens, wie es in Figur 6 dargestellt ist, oder
- aus der zwischen den Nukleotiden 579 und 1092 der Figur 6 enthaltenen Sequenz, oder
- aus der zwischen den Nukleotiden 912 und 1092 der Figur 6 enthaltenen Sequenz
- aus jeder Sequenz, die von einer der vorhergehenden Sequenzen durch Addition, Deletion oder Substitution von Nukleotiden ohne wesentliche Modifizierung der Expression des heterologen Proteins abgeleitet ist.

3. Expressionssystem nach Anspruch 1 oder 2, **dadurch gekennzeichnet, dass** die Verstärkersequenz der Enhancer des murinen CD4-Gens ist und die gesamte oder einen Teil der folgenden Sequenz in Form einer oder mehrere Kopien umfasst:

TGTTGGGGTT CAAATTTGAG CCCCAGCTGT TAGCCCTCTG CAAAGAAAAA
AAAAAAAAAA AAAGAACAAA GGGCCTAGAT TTCCCTTCTG AGCCCCACCC
TAAGATGAAG CCTCTTCTTT CAAGGGAGTG GGGTTGGGGT GGAGGCGGAT
CCTGTCAGCT TTGCTCTCTC TGTGGCTGGC AGTTTCTCCA AAGGGTAACA
GGTGTCAGCT GGCTGAGCCT AGGCTGAACC CTGAGACATG CTACCTCTGT
CTTCTCATGG CTGGAGGCAG CCTTTGTAAG TCACAGAAAG TAGCTGAGGG
GCTCTGGAAA AAAGACAGCC AGGGTGGAGG TAGATTGGT

4. Expressionssystem nach einem der Ansprüche 1 bis 3, **dadurch gekennzeichnet, dass** der Vektor außerdem in Abhängigkeit vom CD4-Promotor oder eines seiner Derivate die gesamte oder einen Teil der cDNA des humanen CD4-Gens enthält.

5. Expressionssystem nach einem der Ansprüche 1 bis 4, **dadurch gekennzeichnet, dass** der Vektor außerdem einen Expressions-Silencer des CD8-Gens enthält.

6. Expressionssystem nach einem der Ansprüche 1 bis 5, **dadurch gekennzeichnet, dass** das heterologe Protein, das geeignet ist, in den reifen T-Lymphozyten exprimiert zu werden, eine konditionelle Toxizität für den reifen T-Lymphozyten aufweist.

7. Expressionssystem nach Anspruch 6, **dadurch gekennzeichnet, dass** das heterologe Protein HSV1-TK Thymidinkinase ist oder ein davon abgeleitetes Protein, das geeignet ist, den Tod des aktivierten T-Lymphozyten in Gegenwart eines Nukleotid-Analogen wie Aciclovir oder Ganciclovir hervorzurufen.

8. Expressionssystem nach einem der Ansprüche 1 bis 7, **dadurch gekennzeichnet, dass** der Vektor entweder
- ein retroviraler Vektor ist und wobei der Enhancer und gegebenenfalls der von dem humanen CD4-Promotor stammende Promotor in die zwei LTR-Sequenzen des retroviralen Vektors inseriert wurden, oder
- ein adenoviraler Vektor oder ein AAV-Vektor ist, oder
- ein nicht-biologisches System, welches Nukleinsäuren freisetzt.

9. Zelle der hämapoietischen Linie, die mit einem Expressionsvektor transfiziert ist, bestehend aus wenigstens einer Sequenz, die ein heterologes Protein codiert, aus einem Promotor und aus einer Polyadenylierungssequenz, **dadurch gekennzeichnet, dass** der Vektor wenigstens eine Verstärkersequenz eines Säuger-CD4-Gens der gleichen Spezies oder einer anderen Spezies enthält, wobei das heterologe Protein hauptsächlich in den reifen T-Lymphozyten exprimiert wird, die aus der Repertoire-Auswahl stammen.

10. Transfizierte Zelle nach Anspruch 9, **dadurch gekennzeichnet, dass** der Vektor einen Promotor enthält. der aus einer der folgenden Sequenzen besteht:
- aus dem Promotor eines CD4-Gens, insbesondere aus dem Promotor des humanen CD4-Gens, wie es in Figur 6 dargestellt ist, oder
- aus der zwischen den Nukleotiden 579 und 1092 in der Numerierung der Figur 6 enthaltenen Sequenz, oder
- aus der zwischen den Nukleotiden 921 und 1092 in der Numerierung der Figur 6 enthaltenen Sequenz, oder
- aus jeder Sequenz, die von einer der vorhergehenden Sequenzen durch Addition, Deletion oder Substitution von Nukleotiden ohne wesentliche Modifizierung der Expression des heterologen Proteins abgeleitet ist.

11. Transfizierte Zelle nach einem der Ansprüche 9 bis 10, **dadurch gekennzeichnet, dass** das heterologe Protein eine konditionelle Toxizität für reife T-Zellen aufweist.

12. Transfizierte Zelle nach einem der Ansprüche 9 bis 11, **dadurch gekennzeichnet, dass** das heterologe Protein HSV1-TK Thymidinkinase ist oder ein davon abgeleitetes Protein, welches geeignet ist, den Tod des aktivierten T-Lymphozyten in Gegenwart eines Nukleotid-Analogen wie Aciclovir oder Ganciclovir hervorzurufen.

13. Verwendung eines Expressionssystems nach einem der Ansprüche 1 bis 8 zur Herstellung eines Medikamentes zur Verwendung in der Gentherapie, um spezifisch die aktivierten T-Lymphozyten zu zerstören.

14. Verwendung nach Anspruch 13 zur Herstellung eines Medikamentes zur Vorbeugung der Abstoßung eines Implantats oder der Abstoßungsreaktion gegen den Wirt.

15. Verwendung nach Anspruch 13 zur Herstellung eines Medikaments zur Vorbeugung oder zur Behandlung von Virusinfektionen, insbesondere von HIV.

16. Verwendung nach Anspruch 13 zur Herstellung eines Medikaments zur Vorbeugung oder zur Behandlung von Autoimmunkrankheiten.

17. Verwendung nach Anspruch 13 zur Herstellung eines Medikaments zur Vorbeugung oder zur Behandlung von einfachen oder sekundären Immundefiziten.

18. Therapeutische Zusammensetzung zur Vorbeugung der Abstoßung eines Implantats, **dadurch gekennzeichnet, dass** sie ein Expressionssystem eines Transgens nach einem der Ansprüche 1 bis 8 enthält.

19. Therapeutische Zusammensetzung zur Vorbeugung oder zur Behandlung von Virusinfektionen, insbesondere von HIV, **dadurch gekennzeichnet, dass** sie ein Expressionssystem eines Transgens nach einem der Ansprüche 1 bis 8 enthält.

20. Therapeutische Zusammensetzung zur Vorbeugung oder zur Behandlung von Autoimmunkrankheiten, **dadurch gekennzeichnet, dass** sie ein Expressionssystems eines Transgens nach einem der Ansprüche 1 bis 8 enthält.

21. Therapeutische Zusammensetzung zur Vorbeugung oder zur Behandlung der Abstoßungsreaktion gegen den Wirt, **dadurch gekennzeichnet, dass** sie ein Expressionssystems eines Transgens nach einem der Ansprüche 1 bis 8 enthält.

22. Verwendung einer Verstärkersequenz eines CD4-Gens, insbesondere des humanen Gens, zur Herstellung eines Medikaments, das die spezifische Expression eines Transgens in reifen T-Lymphozyten erlaubt.

23. Verwendung nach Anspruch 22 einer Verstärkersequenz und eines Promotors eines CD4-Gens.

## Claims

1. System for expressing a protein or a heterologous gene, which system comprises a recombinant vector which can be used for transducing cells of the hematopoietic lineage, in particular stem cells, such that said cell which has been thus transduced will only express the protein or the heterologous gene in mature T cells after repertoire selection, said vector being provided with all the sequences which are required for its expression, and **characterized in that** it contains at least one enhancer of a CD4 gene of the same species or of a different species.

2. Expression system according to claim 1, **characterized in that** the vector also comprises a promoter which consists of one of the following sequences:
- the promoter of a CD4 gene, in particular the pomoter of the human CD4 gene, as depicted in Figure 6, or
- the sequence contained between nucleotides 579 to 1092 of Figure 6, or
- the sequence contained between nucleotides 912 to 1092 of Figure 6
- any sequence which is derived from one of the preceding sequences by the addition, deletion or substitution of nucleotides without substantial modification of the expression of the heterologous protein.

3. Expression system according to claim 1 or 2, **characterized in that** the enhancer sequence is the murine CD4 enhancer and comprises all or part of the following sequence in the form of one to several copies:

TGTTGGGGTT CAAATTTGAG CCCCAGCTGT TAGCCCTCTG CAAAGAAAAA
AAAAAAAAAA AAAGAACAAA GGGCCTAGAT TTCCCTTCTG AGCCCCACCC
TAAGATGAAG CCTCTTCTTT CAAGGGAGTG GGGTTGGGGT GGAGGCGGAT
CCTGTCAGCT TTGCTCTCTC TGTGGCTGGC AGTTTCTCCA AAGGGTAACA
GGTGTCAGCT GGCTGAGCCT AGGCTGAACC CTGAGACATG CTACCTCTGT
CTTCTCATGG CTGGAGGCAG CCTTTGTAAG TCACAGAAAG TAGCTGAGGG
GCTCTGGAAA AAAGACAGCC AGGGTGGAGG TAGATTGGT

4. Expression system according to one of claims 1 to 3, **characterized in that** the vector further contains, under the control of the CD4 promoter or one of its derivatives, all or part of the cDNA of the human CD4 gene.

5. Expression system according to one of claims 1 to 4, **characterized in that** the vector further contains a silencer of the expression of the CD8 gene.

6. Expression system a cording to one of claims 1 to 5, **characterized in that** the heterologous protein which is capable of being expressed in mature T lymphocytes exhibits conditional toxicity for the mature T lymphocyte.

7. Expression system according to claims 6, **characterized in that** the heterologous protein is HSV1-TK thymidine kinase or a protein derived from this kinase, which is capable of inducing the death of the activated T lymphocyte in the presence of a nucleotide analog such as acyclovir or ganciclovir.

8. Expression system according to one of claims 1 to 7, **characterized in that** the vector is either:
- a retroviral vector, with the enhancer and, where appropriate, the promoter derived from the human CD4 promoter being inserted between the two LTR sequences of the retroviral vector,
- an adenoviral vector or an AAV vector, or
- a non-biological system for delivering nucleic acids.

9. Cell of the hematopoietic lineage which is transfected with an expression vector which consists at least of a sequence encoding a heterologous protein, a promoter and a polyadenylation sequence, **characterized in that** the said vector contains at least one enhancer sequence from a mammalian CD4 gene of the same species or of a different species, with the heterologous protein being principally expressed in mature T lymphocytes which are derived from repertoire selection.

10. Transfected cell according to claim 9, **characterized in that** the vector contains a promoter which consists of one of the following sequences:
- the promoter of a CD4 gene, in particular the promoter of the human CD4 gene, as depicted in Figure 6, or
- the sequence contained between nucleotides 579 to 1092 in the numeration of Figure 6, or
- the sequence contained between nucleotides 912 to 1092 in the numeration of Figure 6, or
- any sequence which is derived from one of the preceding sequences by the addition, deletion or substitution of nucleotides without substantial modification of the expression of the heterologous protein.

11. Transfected cell according to one of claims 9 to 10, **characterized in that** the heterologous protein exhibits conditional toxicity for mature T cells.

12. Transfected cell according to one of claims 9 to 11, **characterized in that** the heterologous protein is HSV1-TK thymidine kinase or a protein derived from this kinase, which is capable of inducing the death of the activated T lymphocyte in the presence of a nucleotide analog such as acyclovir or ganciclovir.

13. Use of an expression system according to one of claims 1 to 8, in the manufacture of a medicament which can be employed in gene therapy for specifically destroying activated T lymphocytes.

14. Use according to claim 13, in the manufacture of a medicament to prevent graft rejection or graft-versus-host disease.

15. Use according to claim 13, in the manufacture of a medicament to prevent or treat viral infections, in particular due to HIV.

16. Use according to claim 13, in the manufacture of a medicament to prevent or treat autoimmune diseases.

17. Use according to claim 13, in the manufacture of a medicament to prevent or treat primary or secondary immune deficiencies.

18. Therapeutic composition which is intended for preventing graft rejection, **characterized in that** it comprises a transgene expression system according to one of claims 1 to 8.

19. Therapeutic composition which is intended for preventing or treating viral infections, particularly infections due to HIV, **characterized in that** it comprises a transgene expression system according to one of claims 1 to 8.

20. Therapeutic composition which is intended for preventing or treating autoimmune diseases, **characterized in that** it comprises a transgene expression system according to one of claims 1 to 8.

21. Therapeutic composition which in intended for preventing or treating graft-versus-host disease, **characterized in that** it comprises a transgene expression system according to one of claims 1 to 8.

22. Use of a CD4 gene enhancer sequence, in particular a human CD4 gene enhancer sequence, in the manufacture of a medicament allowing expression of a transgene specifically in mature T lymphocytes.

23. Use according to claim 22, of an enhancer sequence and a promoter of a CD4 gene.
